# EUROPEAN PATENT SPECIFICATION

(11) **EP 0 487 758 B1**
(45) Date of publication and mention of the grant of the patent: **05.03.1997**
(21) Application number: 90122541.7
(22) Date of filing: 26.11.1990
(51) Int. Cl.: A61F 13/58

(54) **Disposable diapers**
Wegwerfbare Windel
Couche à jeter

(43) Date of publication of application: 03.06.1992
(73) Proprietor: Toyo Eizai Kabushiki Kaisha, Kawanoe-shi Ehime-ken799-01 (JP)
(72) Inventor: Uda, Masashi, Minoo-shi, Osaka 562 (JP)
(74) Representative: TER MEER - MÜLLER - STEINMEISTER & PARTNER

(56) References cited:
- EP-A- 0 080 647
- EP-A- 0 249 073
- FR-A- 2 137 855
- FR-A- 2 515 004
- FR-A- 2 552 662
- FR-A- 2 586 558
- US-A- 3 920 018

## Description

The present invention relates to a disposable diaper comprising a water permeable sheet and an absorbent and a non water permeable sheet, comprising two web members disposed on opposite sides of a first longitudinal end of the diaper body, the web members being capable to be connected around the wearer's body for preliminarily fixing the diaper on the wearer with the first longitudinal end of the diaper being positioned on the back of the wearer, and fastening means at both sides of the second longitudinal end of the diaper body for closing the diaper on the front side of the wearer's body.

Various kinds of disposable diapers made from water permeable sheets, abosrbing materials and non-water permeable sheets have been already commercially available. Most of these commercially available diapers have a construction that a pair of fastening tapes are secured to both sides of one end side (generally on the back side) in a longitudinal direction of a diaper body, and the fastening tapes are adherred to a surface sheet (a non-water permeable sheet) on the other end side (generally on the stomach or inside) of the diaper body by making use of pressure-sensitive adhesives or the like attached to the fastening tapes so that the diaper is fitted.

However, in such a disposable diaper as mentioned above, the fastening tapes cannot be adherred unless a wearer is upwardly laid down. That is, a wearer such as an infant or baby is laid down, the disposable diaper is placed under the hip, one end of the disposable diaper is made to pass through the thigh into contact with the stomach, and the diaper is formed into shorts. In the case where an attempt is made to fit a diaper on the body of a wearer while standing upright, it is impossible to stop the fastening tapes while holding the diaper applying it to the thigh by two hands, resulting in inconveniences that the diaper is dropped down, the fastening tapes cannot be fitted while stretching them so that they come into intimate contact with the body portion, and the diaper cannot be closely fitted on the thigh portion.

To comply with these difficulties, the FR-A-2 586 558 discloses a sanitary napkin or the like having webs on both sides of a first longitudinal end which may be closed around the body of the wearer, while the napkin body is hanging down on the back of the wearer. After closing and fixing the webs, the napkin is drawn to the frontside between the legs of the wearer and attached to the web by means of adhesive spots provided on the inner surface of the second longitudinal end.

There is another problem that when the wearer moves while the diaper is being fitted, the diaper tends to be deviated in position. That is, since the disposable diaper stopped by the fastening tapes is merely adherred in a point-like fashion on the left and right hands of the stomach, there was an inconvenience that the deviation of position tends to occur by movement of the wearer. For solving the problem as mentioned above, a diaper is commercially available in which elastic yarns or tape-like elastic members are mounted on the ends in a longitudinal direction of the disposable diaper so that the body portion is elastically held by the elastic yarns or the like. However, it is not possible to completely prevent the end of the diaper body from occurrence of deviation in position about the stopped portion of the fastening tapes with the result that urine or soft stools often leak out of the thigh or the like of the body.

It is a first object of the present invention to provide a diaper which can be fitted in the attitude of a standing wearer. A second object of the invention is to provide a disposable diaper which can minimize occurrence of a deviation of position when the diaper is worn to positively prevent urine or the like from leaking. Another object of the invention is to provide a disposable diaper which can be used without producing a feeling of pressure and a feeling of displeasure.

To comply with the objects of the invention, the diaper according to the present invention is characterized in that the fastening means comprise adhesive tapes comprising a pressure sensitive adhesive and being adapted to be attached to the outer surface of the first longitudinal end of the diaper, in that at least one of said web members has an expansible elastic portion, in that one web member is formed at the extreme end with a tacky layer and the other web member is formed with a release portion having a narrower width W₂ than the width W₁ of the web member, and in that the web members are fixed in a position deviated downwardly from the end edge of the diaper body by a length y which is substantially the same as the width of the fastening tapes.

Thus, a diaper according to the present invention has, on one longitudinal end, the usual, known fastening tapes and on the other longitudinal end two web members which, when the diaper is attached to the body of a wearer, are connected while being urged against the stomach portion of the wearer. In this state, the diaper body is temporarily held on the body of the wearer.

Embodiments of the invention are described in the following with reference to the drawings.

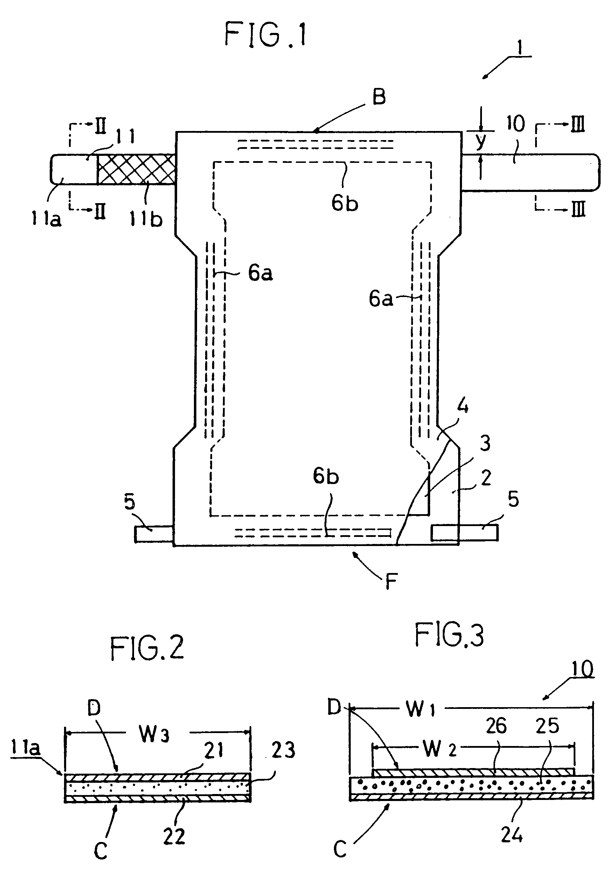

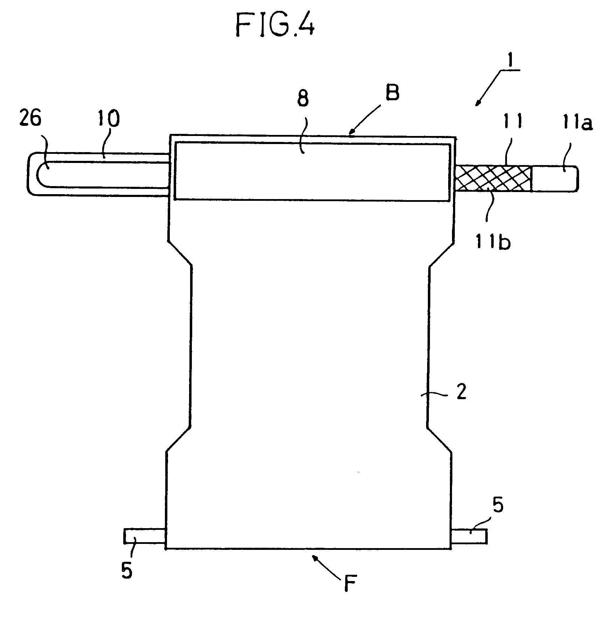

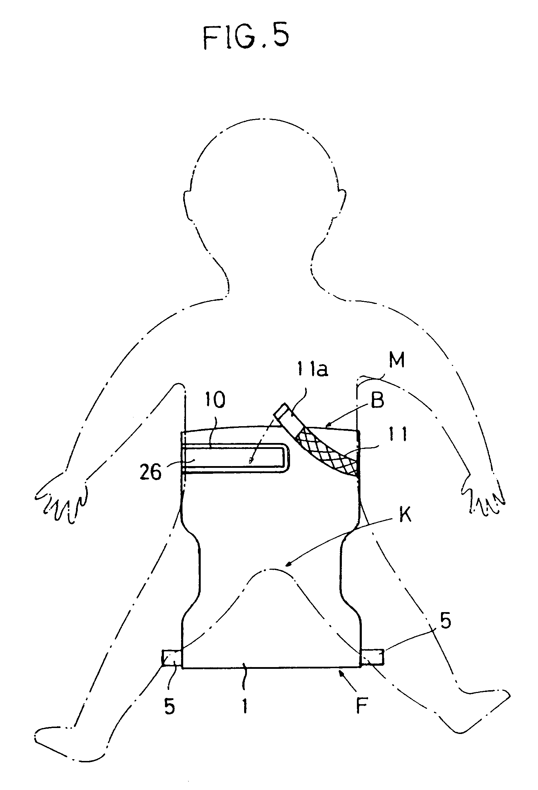

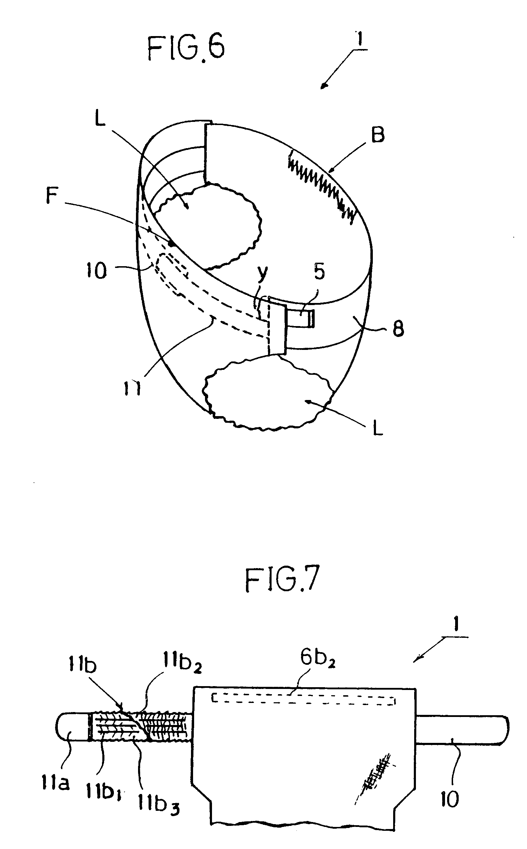

FIG. 1 is a plan view showing a typical embodiment of the present invention; FIG. 2 is a sectional view taken on line II-II of FIG. 1; FIG. 3 is a sectional view taken on line III-III of FIG. 1; FIG. 4 is a rear view of FIG. 1; FIG. 5 is an explanatory view showing the fitting process of the disposable diaper shown in FIG. 1; FIG. 6 is an explanatory view showing the fitting state of the disposable diaper shown in FIG. 1; and FIG. 7 is an explanatory view showing another embodiment of the present invention.

FIG. 1 is a plan view showing a typical embodiment of the present invention. A diaper body 1 is formed from a water permeable sheet 4, an absorbent 3, a non-water permeable sheet 2 and so on. Peripheral edge portions of the water permeable sheet 4 and the non-water permeable sheet 2 are adherred to each other to form a bag body into which is received the absorbent 3 formed of high water absorptive resin, crushed pulp, tissue or the like. If required, in parts corresponding to the thigh, the stomach, and the back, yarn-like or sheet-like elastic members 6a and 6b are put between the sheets 2 and 4 to form an expansible gathered portion.

A pair of known fastening tapes 5 with one side thereof coated with a pressure sensitive adhesive are mounted on both sides of one end in a longitudinal direction of the diaper body 1. The mounting side of the fastening tape 5 corresponds to the stomach side F of a wearer conversely to that of conventional goods when a diaper is worn.

A pair of web members 10 and 11 are secured to both sides of the other end corresponding to the back side B of the diaper body 1. At least one of the web members is provided with an elastic portion. In the illustrated example, an expansible non-woven fabric (a sole elastic fiber or a non-woven fabric made by mixing the former) as an elastic portion 11b is used for a part of the web member 11, and an adhesive portion 11a is formed at the extreme end thereof. The adhesive portion 11a is formed by laminating a tacky layer 23 such as pressure sensitive adhesives on a film-like substrate 21 and attaching a release tape 22 to the tacky layer 23. If the release tape 22 is peeled off, it can be attached to a release portion 26 of the web member 11 as will be described later. Reference character C denotes the skin surface side of a wearer, and D denotes the outer surface side. It is to be noted that the aforesaid elastic portion 11a may be in the form of a gathered portion in which a yarn-like or tape-like elastic body 11b₁ is added in an elongated state to a non-woven fabric 11b₂ or a film 11b₃ or in the form of a sheet-like synthetic resin foamed body having an elasticity, as shown in FIG. 7.

The other web member 10 is formed by laminating a synthetic resin foamed sheet 25 on a non-woven fabric 24 and laminating a synthetic resin film as a release portion 26 thereon, as shown in FIG. 3, and the outer surface side D of the synthetic resin film employed has a peelability and strength such that adhesion and disengagement of the tacky layer 23 can be carried out repeatedly. The peripheral edge corner of the web member 10 is formed to have a smooth curve, and the release portin 26 preferably has a width W₂ narrower than a width W₁ of the foamed sheet 25 or the like, whereby preventing the edge of the synthetic resin film from directly contacting the skin surface of the wearer to prevent an unpleasant feeling such as pain from occurrence. The material of the release portion 26 is not limited to the synthetic resin film but may be those in which the non-woven fabric or cloth is caoted with well-known peeling agent or the like. It is further noted that the foamed sheet 25 may be omitted or may be replaced by other bulky non-woven fabrics or clothes having a flexibility. Further, connection means for the web members may be of fragmentary adhesive tapes, or those in which both the web members are coated with self-adhesive tacky agents or other connection means such as perchlotapes or those in which one of the web members 10 and 11 is formed to be longer and the other is provided integrally on the surface of the diaper body 1 so that the body portion is substantially encircled by a single web member.

FIG. 4 is a rear view of FIG. 1. A synthetic resin film 8 is attached to the end of the back side B of the diaper body 1 so that the adhesion and disengagement of the fastening tape 5 are preferably effected repeatedly.

Preferably, the web members 10 and 11 are fixed while being deviated downwardly by a length y from the end edge of the diaper body 1 as shown in FIG. 1, whereby as will be described in detail later, the connected web members 10 and 11 are fixed in a lower position than a ring-like fixing means formed by the fastening tape 5 to minimize the deviation of position of the diaper body 1. It is suggested that the length y be substantially the same as the width of the fastening tape 5.

Next, an example of wearing a disposable diaper shown in the above-described embodiment will be described with reference to FIGS. 5 and 6. First, the web members 10 and 11 are wound about the body portion of a wearer W as shown in FIG. 5. At this time, the adhesive portion 11a is attached to the upper surface of the release portion 26 of the web member 10 while applying and urging the web member 10 against the stomach portion by one hand and slightly drawing out the elastic portion 11b of the web member 11b by the other hand. In this state, the diaper body 1 is temporarily stopped to the body portion of the wearer by the web members 10 and 11 and hung on the hip portion. Therefore, the stomach portion F of the diaper body 1 is made to pass through the thigh K to be guided to the stomach side and the fastening tapes 5 and 5 are attached to the film 8 on the outer surface on the other side of the diaper. The diaper is used in the worn state shown in FIG. 6.

The method of wearing the diaper is not limited to the example described above but the method can be employed in which the stomach and back sides of the wearer may be reversed or the diaper may be applied to the stomach side and temporarily stopped, after which the diaper is deviated through 180° and the fastening tapes 5 and 5 may be used in a manner similar to that as described above to wear the diaper.

As described above, the disposable diaper according to the present invention can be fitted not only in the state where a wearer is laid down but also in the state where a wearer is standing upright. In addition, since a part of the web members is expansible, the wearer does not feel pressure caused by the web members 10 and 11. Furthermore, since the skin surface side of the web member 10 is formed of a soft foamed sheet or the like, the wearer does not feel pain caused by the web members 10 and 11.

The disposable diaper of the present invention can be applied not only to the example as described above but also to all kinds of well-known disposable diapers such as those in which a gathered portion is not formed on the thigh portion or those in which fastening tapes are of expansible.

The present invention is configured as described above. Therefore, the web members are used as members for temporary stopping whereby the diaper can be fitted on a stood-up wearer. Furthermore, the web member is partly formed with an elastic portion and a flexible foamed sheet is used, thus inducing no displeasure feeling such as pressure, pains or the like.

Furthermore, since the diaper body can be fitted in close contact with the wearer by both the web members and fastening tapes, the diaper becomes hard to be deviated in position during wearing even if the wearer is greatly moved.

## Claims

1. A disposable diaper comprising a water permeable sheet and an absorbent and a non-water permeable sheet, comprising two web members disposed on opposite sides of a first longitudinal end of the diaper body, the web members (10,11) being capable to be connected around the wearer's body for preliminarily fixing the diaper on the wearer with the first longitudinal end of the diaper being positioned on the back of the wearer, and fastening means at both sides of the second longitudinal end of the diaper body for closing the diaper on the front side of the wearer's body, **characterized** in that the fastening means comprise adhesive tapes (5) comprising a pressure sensitive adhesive and being adapted to be attached to the outer surface of the first longitudinal end of the diaper, in that at least one of said web members (10 or 11) has an expansible elastic portion (11b), in that one web member (11) is formed at the extreme end with a tacky layer (23) and the other web member (10) is formed with a release portion (26) having a narrower width (W₂) than the width (W₁) of the web member (10), and in that the web members (10,11) are fixed in a position deviated downwardly from the end edge of the diaper body (1) by a length (y) which is substantially the same as the width of the fastening tapes (5).

2. A disposable diaper according to claim 1, **characterized** in that said elastic portion (11b) is formed from an expansible non-woven fabric.

3. A disposable diaper according to claim 1, **characterized** in that said elastic portion (11b) is formed by adding an elongated elastic body to a non-woven cloth or synthetic resin film.

4. A disposable diaper according to claim 3, **characterized** in that a release tape (22) is attached to said tacky layer.

5. A disposable diaper according to claim 1, **characterized** in that the fastening tape (5) is attached to both sides of the diaper body substantially coincident with the other longitudinal end of the diaper body.

6. A disposable diaper according to claim 1, **characterized** in that an elastic body (6b) is added along the lateral edges of the diaper body (1).

7. A disposable diaper according to claim 1 or 2, **characterized** in that an elastic body (6a) is added along the longitudinal edges of the diaper body (1).

8. A disposable diaper according to claim 1, **characterized** in that a synthetic resin sheet (8) capable of being repeatedly adhered to a tacky agent of the fastening tape (5) is attached to a portion for adhering said fastening tape (5) at the longitudinal end of the diaper on the outer surface side of the non-water permeable sheet of the diaper body (1).

## Patentansprüche

1. Wegwerfwindel mit einer wasserdurchlässigen Schicht und einem Absorptionsmittel und einer wasserundurchlässigen Schicht, mit zwei an entgegengesetzten Seiten eines ersten Längsendes des Windelkörpers angeordneten Laschen (10,11), die um den Körper des Benutzers herum verbindbar sind, um die Windel vorläufig am Benutzer zu befestigen, wobei das erste Längsende der Windel am Rücken des Benutzers angeordnet ist, und Befestigungsmitteln an beiden Seiten des zweiten Längsendes des Windelkörpers, zum Schließen der Windel an der Vorderseite des Körpers des Benutzers, dadurch **gekennzeichnet**, daß die Befestigungsmittel Klebestreifen (5) mit einem druckempfindlichen Kleber aufweisen und dazu ausgebildet sind, an der äußeren Oberfläche des ersten Längsendes der Windel angebracht zu werden, daß wenigstens eine der Laschen (10) oder (11) einen dehnbaren elastischen Teil (11b) hat, daß eine Lasche (11) am äußeren Ende mit einer klebrigen Schicht und die andere Lasche (10) mit einem Löseteil (26) versehen ist, das eine geringere Breite (W₂) als die Breite (W₁) der Lasche (10) hat, und daß die Laschen (10,11) in einer Position befestigt sind, die vom stirnseitigen Rand des Windelkörpers (1) um eine Länge (y) nach unten versetzt ist, die im wesentlichen gleich der Breite der Befestigungsstreifen (5) ist.

2. Wegwerfwindel nach Anspruch 1, dadurch **gekennzeichnet**, daß der elastische Teil (11b) aus einem dehnbaren, ungewebten Textilmaterial gebildet ist.

3. Wegwerfwindel nach Anspruch 1, dadurch **gekennzeichnet**, daß der elastische Teil (11b) durch Hinzufügen eines länglichen elastischen Körpers zu einem ungewebten Stoff oder einer Kunststoffolie gebildet ist.

4. Wegwerfwindel nach Anspruch 3, dadurch **gekennzeichnet**, daß ein Lösestreifen (22) an der klebrigen Schicht angebracht ist.

5. Wegwerfwindel nach Anspruch 1, dadurch **gekennzeichnet**, daß der Befestigungsstreifen (5) an beiden Seiten des Windelkörpers im wesentlichen in Höhe des anderen Längsendes des Windelkörpers angebracht ist.

6. Wegwerfwindel nach Anspruch 1, dadurch **gekennzeichnet**, daß ein elastischer Körper (6b) längs der Querränder des Windelkörpers (1) angebracht ist.

7. Wegwerfwindel nach Anspruch 1 oder 2, dadurch **gekennzeichnet**, daß ein elastischer Körper (6a) längs der Längsränder des Windelkörpers (1) angebracht ist.

8. Wegwerfwindel nach Anspruch 1, dadurch **gekennzeichnet**, daß ein Kunststoffblatt (8), das wiederholt an ein klebriges Mittel des Befestigungsstreifens (5) angeklebt werden kann, an einem Teil zur Anbringung des Befestigungsstreifens (5) am Längsende der Windel auf der Seite der äußeren Oberfläche der wasserundurchlässigen Schicht des Windelkörpers (1) angebracht ist.

## Revendications

1. Couche jetable comprenant une feuille perméable à l'eau, une matière absorbante et une feuille non perméable à l'eau, comportant deux éléments de bande disposés sur les côtés opposés d'une première extrémité longitudinale du corps de couche, les éléments de bande (10, 11) pouvant être reliés autour du corps du porteur pour fixer préalablement la couche sur le porteur avec la première extrémité longitudinale de la couche disposée sur le dos du porteur, et des moyens de fixation situés sur les deux côtés de la seconde extrémité longitudinale du corps de couche et servant à fermer la couche sur la face avant du corps du porteur, caractérisée en ce que les moyens de fixation comprennent des rubans adhésifs (5) comportant une substance auto-adhésive et agencés de façon à être attachés à la surface extérieure de la première extrémité longitudinale de la couche, en ce qu'au moins l'un des éléments de bande (10 ou 11) comporte une partie élastique extensible (11b), en ce qu'un premier élément de bande (11) est pourvu à son extrémité extérieure d'une couche collante (23) et le second élément de bande (10) est pourvu d'une partie antiadhésive (26) ayant une largeur (W₂) plus étroite que la largeur (W₁) de l'élément de bande (10) et en ce que les éléments de bande (10, 11) sont fixés dans une position décalée vers le bas, à partir du bord d'extrémité du corps de couche (1), d'une longueur (y) qui est pratiquement la même que la largeur des rubans de fixation (5).

2. Couche jetable selon la revendication 1, caractérisée en ce que la partie élastique (11b) est formée d'un tissu non tissé extensible.

3. Couche jetable selon la revendication 1, caractérisée en ce que la partie élastique (11b) est formée en ajoutant un corps élastique allongé à une toile non tissée ou un film de résine synthétique.

4. Couche jetable selon la revendication 3, caractérisée en ce qu'un ruban antiadhésif (22) est solidaire de la couche collante.

5. Couche jetable selon la revendication 1, caractérisée en ce qu'un ruban de fixation (5) est fixé aux deux côtés du corps de couche d'une manière coïncidant pratiquement avec la seconde extrémité longitudinale du corps de couche.

6. Couche jetable selon la revendication 1, caractérisée en ce qu'un corps élastique (6b) est ajouté le long des bord latéraux du corps de couche (1).

7. Couche jetable selon la revendication 1 ou 2, caractérisée en ce qu'un corps élastique (6a) est ajouté le long des bords longitudinaux du corps de couche (1).

8. Couche jetable selon la revendication 1, caractérisée en ce qu'une feuille de résine synthétique (8) apte à être collée d'une manière répétée sur un agent collant du ruban de fixation (5) est solidaire d'une partie servant à coller le ruban de fixation (5) et située à l'extrémité longitudinale de la couche sur la face superficielle extérieure de la feuille non perméable à l'eau du corps de couche (1).
